# EUROPEAN PATENT APPLICATION

(11) **EP 4 763 162 A1**
(43) Date of publication of application: **24.06.2026**
(21) Application number: 25222771.5
(22) Date of filing: 11.12.2025
(51) Int. Cl.: A61F 11/08, A61F 11/12

(54) **EARPLUG**

(30) Priority: 20.12.2024 SE 2451327
(71) Applicant: SwedSafe AB, 282 35 Tyringe (SE)
(72) Inventor: Berg, Göran, 282 33 Tyringe (SE)
(74) Representative: Hansson Thyresson AB

(57) **Abstract**

The present invention relates to a stem (10) for an earplug (50), an earplug comprising said stem, and a hearing protection system (60) comprising at least two earplugs, each having a stem. The stem includes a proximal portion (12) including an attachment portion (16) arranged for attachment to a sound attenuating element (52), a distal portion (14) including an engaging portion (24) configured to engage with an engagement portion (24) of a stem (10) for another earplug (50).

## Description

### TECHNICAL FIELD

The present invention relates to a stem of an earplug, to an earplug, and to a hearing protecting system including an earplug having a stem.

### BACKGROUND

Various types of hearing protective and/or noise attenuation devices are well known, including, but not limited to earmuffs, semi-aural devices, earplugs etc. Many people prefer earplugs because they are effective in attenuating sound and comfortable and easy to carry with them.

Many earplugs comprise a sound attenuating element that is to be placed in the ear canal of the user to dampen or attenuate sound before reaching inner parts of the ear. The sound attenuating element is commonly made from a resilient, compressible material, e.g., foam or rubber.

Some earplug designs further include a rigid or semi-rigid stem embedded to various degrees in the sound attenuating element. The stem may provide a degree of rigidity to the earplug which facilitates the insertion and removal of the earplug from the ear canal. The stem may also function as a handle for the user to grasp when inserting, removing or adjusting the earplug.

Following from the fact that the earplugs are to be inserted into the ear of a user they generally are small and as such are easily misplaced, dropped or lost. To increase the chances of the earplugs being at hand for the user when needed, in particular in environments where the earplugs are frequently removed from and reinserted into the ear canals of the user, the two earplugs of an earplug pair may be connected to each other using a string, a band, a cord, or other suitable connecting means. By connecting the earplugs by a string, or similar, the earplugs may be kept around the neck of the user when not inserted into the ear canal, thereby reducing the risk of losing one or both of the earplugs and still being readily at hand when needed.

Although the use of a string to connect two earplugs reduces the risk of losing or dropping the earplugs, earplugs are still lost or dropped.

From the above it is understood that there is room for improvements and the invention aims to solve or at least mitigate the above and other problems.

### SUMMARY

One object of the present invention is to further reduce the risk of dropping or losing earplugs.

The invention is defined by the appended independent claims. Additional features and advantages of the concepts disclosed herein are set forth in the description which follows, and in part will be obvious from the description, or may be learned by practice of the described technologies. The features and advantages of the concepts may be realized and obtained by means of the instruments and combinations particularly pointed out in the appended claims. These and other features of the technologies described will become more fully apparent from the following description and appended claims, or may be learned by the practice of the disclosed concepts as set forth herein.

More specifically, according to a first aspect of the invention, a stem for an earplug comprises a proximal portion including an attachment portion arranged for attachment to a sound attenuating element, a distal portion including an engaging portion configured to engage with an engagement portion of a stem for another earplug. One advantage of these features and in particular the engagement portion is that pair of earplugs including stems like these may be attached to each other further reducing the risk of losing earplugs. A further advantage may be experienced in a hearing protection system including cords connecting the pair of earplugs as the risk of entanglement will be reduced. Another advantage may be that the stems formed like this allow users to keep the earplugs at an easy reach.

In some embodiments the distal portion further includes a handle portion arranged for a user to grip. One advantage of the handle portion is that the stem and an earplug including the stem may be handled, inserted into the ear, and adjusted using other protective gear such as gloves. Another advantage may be that the pairing or attaching of two stems may be facilitated as the handle portion will present a tactile guide to the positioning of the stems.

In some embodiments the handle portion includes an edge defining the handle portion, wherein the handle portion has a thickness and a width, and wherein the width of the handle portion is greater than the thickness. Some advantages above relating to the handle portion are that the direction of the handle portion will be more easily noticeable and that the handle portion will be more easily adjusted.

In some embodiments the engaging portion is arranged in the handle portion. This may facilitate the guiding of the user to pair or attach two earplugs to each other.

In some embodiments the engaging portion is formed to allow releasable connection of the stem to the engaging portion of another earplug. This feature may provide the advantage of facilitating temporarily removing hearing protection from the ears of the user with reduced risk of losing them, reduced risk of entanglement, and facilitating the re-insertion of the hearing protection as the earplugs are nearby and not lost or entangled.

In some embodiments the engaging portion is formed as a slot in the stem.

In some embodiments the slot is forming a gap in an edge of the stem, and wherein the slot is wider further from the edge of the stem. This feature has the advantage of being an effective design for having two stems being easily attached to each other and then easily released again when needed. Another advantage is that the design makes the attachment secure until the user willingly separate the two stems from each other.

In some embodiments the slot is extending from the edge towards an opposite side of the stem.

In some embodiments the engaging portion is formed as a slot into the gripping area from the edge, forming a gap at the edge and an opening in the handle portion.

In some embodiments the slot is wider further from the edge of the stem. This feature has the advantage of being an effective design for having two stems being easily attached to each other and then easily released again when needed. Another advantage is that the design makes the attachment secure until the user willingly separate the two stems from each other.

In some embodiments the slot is having an entry cutout at the edge of the handle portion and a retaining cutout, wherein a width of the entry cutout, in a direction along the edge, is smaller than a width of the retaining cutout.

In some embodiments the width of the entry cutout corresponds to the thickness of the handle portion in the area in the vicinity of the slot.

According to another aspect of the invention, an earplug comprises a stem according to any one of the above embodiments and a sound attenuating element attached to the proximal portion of the of the stem. The advantages of this aspect of the invention correspond to the advantages presented in relation to corresponding features presented above.

According to yet another aspect of the invention, a hearing protection system comprises at least two earplugs according to the previously mentioned aspect of the invention, wherein the at least two earplugs are connected to each other by a cord attached to the distal portion of each of the eat least two earplugs. The advantages of this aspect of the invention correspond to the advantages presented in relation to corresponding features presented above.

A further scope of applicability of the present invention will become apparent from the detailed description given below. However, it should be understood that the detailed description and specific examples, while indicating preferred embodiments of the invention, are given by way of illustration only, since various changes and modifications within the scope of the invention will become apparent to those skilled in the art from this detailed description. Hence, it is to be understood that this invention is not limited to the particular component parts of the device described or steps of the methods described as such device and method may vary. It is also to be understood that the terminology used herein is for purpose of describing particular embodiments only, and is not intended to be limiting. It must be noted that, as used in the specification and the appended claim, the articles "a," "an," "the," and "said" are intended to mean that there are one or more of the elements unless the context clearly dictates otherwise. Thus, for example, reference to "an earplug" or "the earplug" may include several earplugs, and the like. Furthermore, the word "comprising" does not exclude other elements or steps.

### BRIEF DESCRIPTION OF THE DRAWINGS

In order to best describe the manner in which the above-described embodiments are implemented, as well as define other advantages and features of the disclosure, a more particular description is provided below and is illustrated in the appended drawings. Understanding that these drawings depict only exemplary embodiments of the invention and are not therefore to be considered to be limiting in scope, the examples will be described and explained with additional specificity and detail through the use of the accompanying drawings in which:
Fig. 1 is a view of a first side of a stem according to some embodiments of the invention,
Fig. 2 is a side view of the stem of Fig. 1,
Fig. 3 is a detailed view of an engaging portion of the stem of Fig. 1,
Fig. 4 is a perspective view of two stems corresponding to the stem of Fig. 1 which are engaging with each other at corresponding engaging portions,
Fig. 5 is a side view of an earplug according to an aspect of the invention, the earplug includes the stem of Fig. 1,
Fig. 6 is a perspective view if a hearing protection system according to an aspect of the invention, the hearing protection system including earplugs corresponding to the earplugs of Fig. 5
Fig. 7 is a perspective view of the hearing protection system of Fig. 6 in which the earplugs are connected at the engaging portions of the stems,
Fig. 8 is a side view of the other side of the stem of Fig. 1 together with a cord not yet connected to the stem,
Fig. 9 is a side view of the same side of the stem as shown in Fig. 1 with the cord now connected to the stem.

Further, in the figures like reference characters designate like or corresponding parts throughout the several figures.

### DETAILED DESCRIPTION

Hereinafter, certain embodiments will be described more fully with reference to the accompanying drawings. It will be apparent to those skilled in the art that various modifications and variations can be made without departing from the inventive concept. Other embodiments will be apparent to those skilled in the art from consideration of the specification and practice disclosed herein. The embodiments herein are provided by way of example so that this disclosure will be thorough and complete, and will fully convey the scope of the inventive concept, and that the claims be construed as encompassing all equivalents of the present inventive concept which are apparent to those skilled in the art to which the inventive concept pertains. If nothing else is stated, different embodiments may be combined with each other.

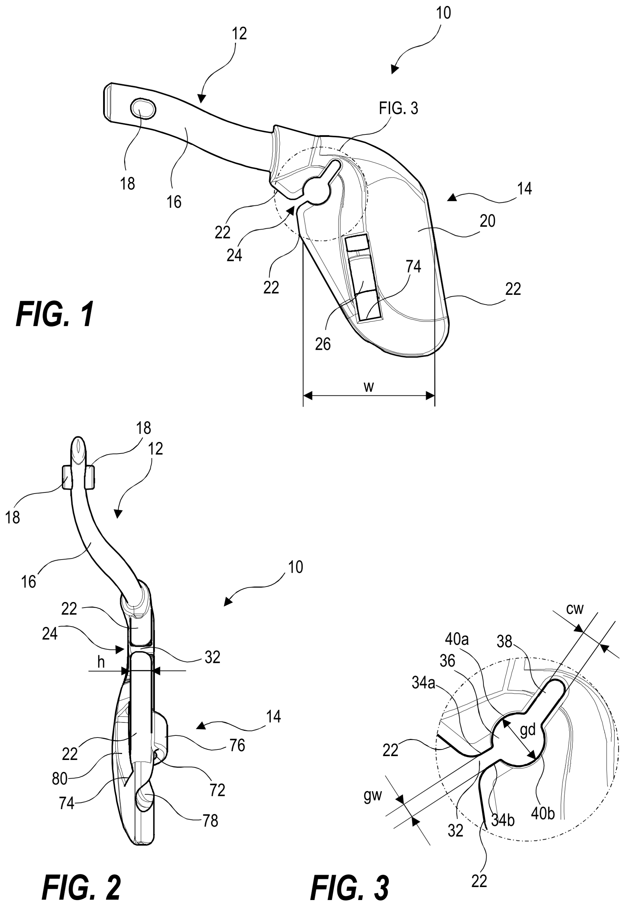

Figs 1 and 2 shows a stem 10 of an earplug according to some embodiments of the invention. The stem 10 generally comprises a proximal portion 12, being the portion of the stem 10 that is entering the ear canal first when the earplug is inserted into the ear canal, and a distal portion 14, being the portion of the stem that is manipulated by the users hand during insertion into, removal from, and/or adjustment of the earplug in the ear canal. The stem may be formed from any suitable plastic material.

The proximal portion 12 includes an attachment portion 16, which is arranged to attach to a sound attenuating element. The attachment portion 16 may have one or a plurality of protrusions 18 formed on its surface for decreasing the risk of the attenuation element coming loose from the attachment portion 16 when mounted.

The distal portion 14 includes a handle portion 20, which is wider than the attachment portion 16 and facilitates gripping and handling of the earplug. The handle portion 20 includes an edge 22 defining the handle portion 20. The edge 22 of the handle portion has a height or thickness h that is less than the width w of handle portion 20. The handle portion further includes an engaging portion 24 and a string connection portion 26.

The engaging portion 24 is a portion of the stem that is configured to engage with a corresponding engaging portion of another earplug. The engaging portion according to some embodiments is formed as a slot in the handle portion. In some embodiments the engaging portion 24 includes alternative engaging means, such as magnets, adhesive, hooks and loops, etc. One advantage of having the engaging portion formed as a slot in the handle portion is that the engaging portion does not need any additional means, devices or substances added to the earplug. The forming of the slot may be achieved already in a mold for forming the stem and in such case the manufacturing process may not require any additional steps to configure or provide the engaging portion 24.

Now referring to Fig. 3 showing an enlarged view of the area of the engaging portion and a slot forming the engaging portion according to some embodiments of the invention. The slot forming the engaging portion includes an opening or a gap 32 in the edge 22 of the handle portion 20. The gap 32 may be seen as defined by gap edges 34. The width gw of the gap may be less than the thickness h of the edge 22 of the handle portion 20 in the area of the gap 32. In some embodiments the width gw is substantially the same as the thickness h of the edge 22. The slot forming the engaging portion 24 is widening after the initial gap through the edge 22 of the handle portion 20, i.e. at a further distance from the edge 22. At this position, i.e. at a further distance from the edge 22, the slot forms a wider through hole 36 through the handle portion 20, i.e. a hole through from one side of the handle portion 20 trough to the other side of the handle portion 20. In some embodiments, like the one showed in Fig. 3, this wider through hole is a round or substantially circular through hole 36 with a diameter gd. Hence the diameter gd is larger than the gap gw, i.e. gd > gw. In the embodiment shown in Fig. 3 the slot continues further into the handle portion 20 as an elongated through hole 38 having a width cw which is smaller than the wider through hole diameter gd.

In other words, the slot described in Fig. 3 may be seen as starting at the edge 22 of the handle portion 20 being defined by two gap edges 34a and 34b running in parallel, in a direction from the edge 22 into the handle portion 20, and at a distance from each other of gw. At a predetermined distance from the edge 22 the distance between the edges 40a and 40b of the slot is increased from the distance gw to the maximum distance of gd as the slot extends further into the handle portion. After the distance between the edges 40a and 40b have reached the maximum distance value gd the distance between the edges 40a and 40b extends further into the handle portion 20 at continuously closer distance from each other in order to form the round or substantially circular through hole 36 of the slot. At a specific distance from the edge 22 where the slot starts the edges 40a and 40b are at a distance of cw from each other and is arranged to continue as parallel edges 42a and 42b at a distance cw from each other further into the handle portion 20 until the slot ends with the two edges forming the slot connecting and forming an inner end of the slot. In the embodiment of Fig. 3 the edges 42a and 42b running at distance cw from each other is running in a direction slightly different from the direction of the edges 34a and 34b defining the slot closer to the edge 22 of the handle portion 20.

According to other embodiments of the stem 10, the edges of a slot of the engaging portion may form other shapes than the one depicted in Figs 1 and 3. One advantages feature that they have in common is that the gap in the edge 22 of the handle portion 20, where the slot starts, is more narrow than the slot further from the edge 22, i.e. the edges forming the slot are closer to each other at the edge of the handle portion than at a predetermined distance into the handle portion, wherein the edges at even further distance from the edge get closer together again to finally meet and form the end of the slot. The slot may be triangular in shape, it may start at the edge 22 with a narrow gap and then expand into a square or into any shape in which the edges of the slot are further apart than at the edge of the handle portion 20.

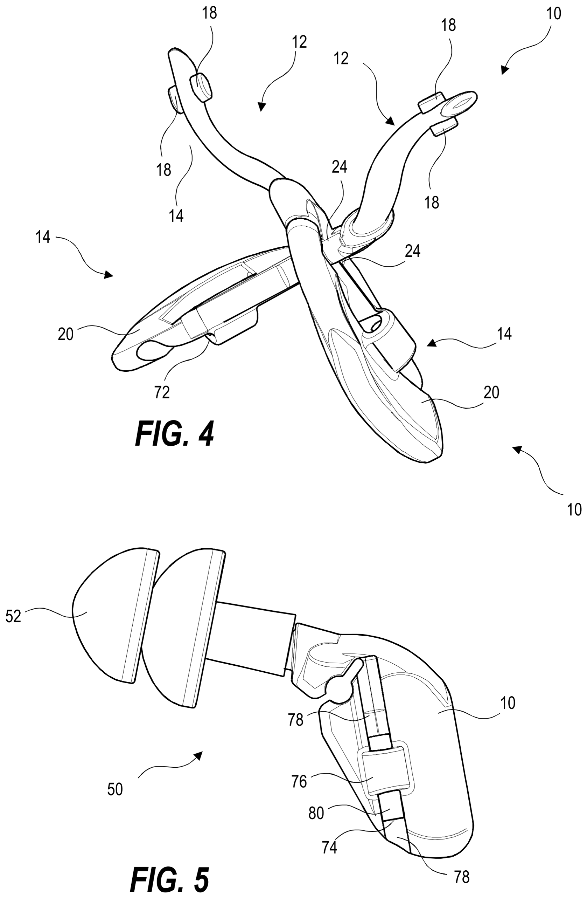

Fig. 4 shows two stems 10, a first stem 10 and a second stem 10, engaging with each other at the engaging portion 24 of each stem 10, respectively. Each of the first and second stems is stems as described in connection with Figs 1-3 By forming the engaging portion 24 as a slot as described above, the two stems may be loosely joined at the engaging portion 24 by bringing the gap 32 in the edge 22 of the handle portion 20 of the first stem 10 to interact with the corresponding engaging portion 24 of the second stem 10 via the gap 32 of the second stem 10. After the two engaging portions 24 have been joined like this the two stems 10 will be loosely joined until the gaps 32 and the initial portion of the corresponding slot is aligned again. When the gaps 32 and the initial portion of the corresponding slot is aligned, the stems 10 may be easily disconnected from each other.

In Fig. 5 an earplug 50 including a stem 10 as described in connection with Figs 1-4 having a sound attenuating element 52 attached to the attachment portion 16 of the stem. The sound attenuating element 52 may be force fit, friction fit or snap-fit, to the attachment portion 16 of the stem 10 or fixed to the attachment portion using, e.g., using adhesive. The sound attenuation element 52 may be formed of a compressible and resilient foam material such as polyvinylchloride (PVC) or polyurethane. Alternatively, the sound attenuation element 52 may be formed from silicone, thermoplastic elastomers, rubber or any other soft formable and resilient material. The sound attenuating element 52 may be preformed and then attached to the stem.

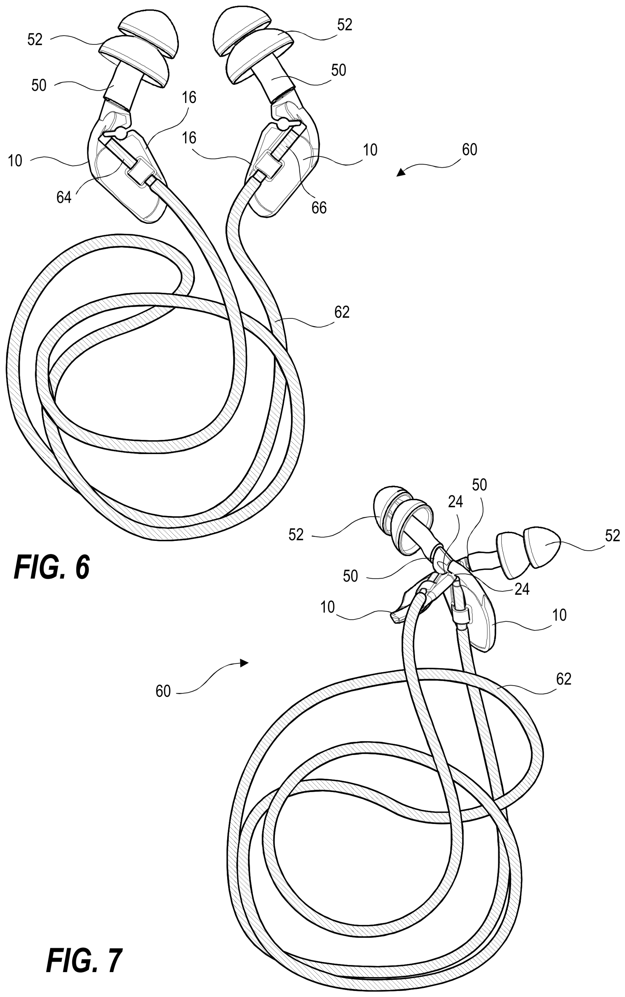

Now referring to Fig. 6, a pair of earplugs 50, as previously described, are forming a hearing protection system 60. The earplugs 50 in the hearing protection system are connected to each other by a cord 62. The cord 62 being a long slender material consisting of one or several strands of material. Hence, the cord 62 may also be referred to as a string, a line, a rope, etc. The material used in the cord may be any material generally used for cords, e.g., polyester, polypropylene, polyethylene, nylon, aramid, poly amide, High Modulus Polyethylene, cotton, jute, Dacron, sisal, etc. The cord 62 includes a first end portion 64 and a second endpoint 66 each being attached to the distal portion 14 of the stem 10 being part of the corresponding earplug 50. By having the earplugs 50 connected to each other via the cord it is possible for a user of the hearing protection system 60 to have the earplugs 50 hanging around the neck when not in use. Therefore, the hearing protection system is always at hand when needed. Moreover, the earplugs 50 includes stems 10 each having the previously described engaging portion 24 and are therefore possible to temporarily attach to each other as shown in Fig. 7. As seen in Fig. 7, the cord and the temporarily attached earplugs 50 form a closed loop of the cord which substantially decreases the risk of the user losing or dropping the hearing protection system 60.

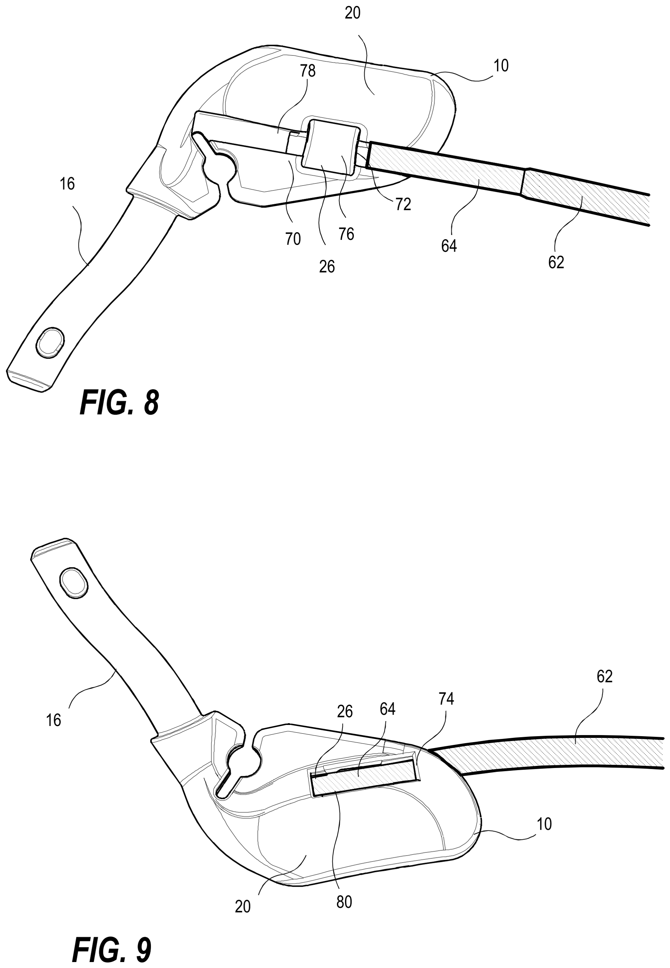

In Figs 8 and 9 a cord 62, according to some embodiments of the invention, is shown together with a stem 10 of an earplug 50. In Fig. 8 the cord 62 is shown before it is connected to the earplug 50 and from a first side of the stem 10. In Fig. 9 the cord 62 and the stem 10 or the earplug 50 is shown when connected and from a second side of the stem 10. Now referring to Fig. 8, the first end portion 64 of the cord 62 is treated to form a rigid end portion 64. This may be achieved by dipping the end portion 64 in a resin, by attaching a sleeve to the end portion 64, rolling a tape around the end portion 64, etc. The second end portion 66 is treated in the same way as the first end portion. Now referring to Figs 8 and 9, the stem 10 includes a cord receiving area 70 configured for receiving the end portion 64, 66, of the cord and to engage with the rigid end portion 64, 66 of the cord 62 for locking it to the cord receiving area 70 of the stem 10 after insertion. The cord receiving area includes an entry opening 72 for receiving the rigid end portion 64, 66 when the cord 62 is to be connected to the stem 10 of the earplug 50. Further, the cord receiving area includes a retraction hindering edge 74, which is configured to stop the cord from being retracted when the rigid end portion as entered the entry opening 72. According, to some embodiments the cord receiving area 70 is configured to angle the rigid end portion 64, 66, after the entire rigid end portion 64, 66, has passed the retraction hindering edge so that the edge of the rigid end portion 64, 66, connecting to the non rigid part of the cord 62 is caught by the retraction hindering edge 74, stopping the rigid end portion 64, 66, from exiting the entry opening 72 and/or from coming loose from the stem. In some embodiments the entry opening 72 is formed by a portion of the handle portion 20 rising in an arc 76 forming an opening between the arc and the material of the handle portion 20 not raised into the arc 76. In some embodiments the material of the handle portion 20 not being the arc and forming the entry opening 72 is part of a groove 78 in the cord receiving area 70 of the handle portion 20. The groove 78 may have a length that exceeds the length of the rigid end portion 64, 66 of the cord. The retraction hindering edge 74 may be formed in the grove by forming a step in the groove 78 at substantially a right angle to a longitudinal direction of the groove. In some embodiments this step is formed by removing the material in the groove so that there is an opening 80 through the handle portion 20 in the groove 78 from the retraction hindering edge 74 and a predetermined distance in a direction coinciding with the direction of inserting the rigid end portion 64, 66, of the cord.

The various embodiments described above are provided by way of illustration only and should not be construed to limit the invention. For example, the principles herein may be applied to any hearing protection system, earplug, and/or stem of an earplug. Those skilled in the art will readily recognize various modifications and changes that may be made to the present invention without following the example embodiments and applications illustrated and described herein, and without departing from the scope of the present disclosure.

## Claims

1. A stem (10) for an earplug (50), comprising:
a proximal portion (12) including an attachment portion (16) arranged for attachment to a sound attenuating element (52),
a distal portion (14) including an engaging portion (24) configured to engage with an engagement portion (24) of a stem (10) for another earplug (50).

2. The stem (10) of claim 1, wherein the distal portion (14) further includes a handle portion (20) arranged for a user to grip.

3. The stem (10) of claim 2, wherein the handle portion (20) includes an edge (22) defining the handle portion (20), wherein the handle portion (20) has a thickness and a width, and wherein the width of the handle portion is greater than the thickness.

4. The stem (10) of any one of claims 2 or 3, wherein the engaging portion (24) is arranged in the handle portion (20).

5. The stem (10) of any one of the preceding claims, wherein the engaging portion (24) is formed to allow releasable connection of the stem (10) to the engaging portion (24) of another earplug (50).

6. The stem (10) of any one of the preceding claims, wherein the engaging portion (24) is formed as a slot (32, 36, 38) in the stem (10).

7. The stem (10) of claim 6, wherein the slot (32, 36, 38) is forming a gap (32) in an edge (22) of the stem (10), and wherein the slot (32, 36, 38) is wider further from the edge (22) of the stem (10).

8. The stem (10) of claim 7, wherein the slot (32, 36, 38) is extending from the edge (22) towards an opposite side of the stem (10).

9. The stem (10) of claim 3, wherein the engaging portion (24) is formed as a slot (32, 36, 38) into the gripping area from the edge (22), forming a gap (32) at the edge and an opening in the handle portion (20).

10. The stem (10) of claim 9, wherein the slot (32, 36, 38) is wider further from the edge (22) of the stem (10).

11. The stem (10) of any one of claims 9 to 10, wherein the slot (32, 36, 38) is having an entry cutout (32) at the edge (22) of the handle portion (20) and a retaining cutout (36), wherein a width of the entry cutout (32), in a direction along the edge, is smaller than a width of the retaining cutout (36).

12. The stem (10) of claim 11, wherein the width of the entry cutout (32) is corresponding to the thickness of the handle portion (20) in the area in the vicinity of the slot (32, 36, 38).

13. An earplug (50), comprising a stem (10) according to any one of claims 1 to 12 and a sound attenuating element (52) attached to the proximal portion (12) of the of the stem (10).

14. A hearing protection system (60), comprising at least two earplugs (50) according to claim 13, wherein the at least two earplugs (50) are connected to each other by a cord (62) attached to a string connection portion (26) of the distal portion (14) of each of the at least two earplugs (50).
